# EUROPEAN PATENT SPECIFICATION

(11) **EP 4 657 450 B1**
(45) Date of publication and mention of the grant of the patent: **24.06.2026**
(21) Application number: 25154965.5
(22) Date of filing: 30.01.2025
(51) Int. Cl.: G16H 30/20

(54) **METHOD AND APPARATUS FOR DISPLAYING SCANNING DATA, DEVICE, AND STORAGE MEDIUM**
VERFAHREN UND VORRICHTUNG ZUR ANZEIGE VON ABTASTDATEN, VORRICHTUNG UND SPEICHERMEDIUM
PROCÉDÉ ET APPAREIL D'AFFICHAGE DE DONNÉES DE BALAYAGE, DISPOSITIF ET SUPPORT D'INFORMATIONS

(30) Priority: 30.05.2024 CN 202410688138
(43) Date of publication of application: 03.12.2025
(73) Proprietor: Shining 3D Tech Co., Ltd., Hangzhou, Zhejiang 311258 (CN)
(72) Inventor: ZHAO, Xiaobo, Hangzhou, 311258 (CN); XIAO, Shujuan, Hangzhou, 311258 (CN); CHEN, Xiaojun, Hangzhou, 311258 (CN); MA, Chao, Hangzhou, 311258 (CN)
(74) Representative: Dehns Germany Partnerschaft mbB

(56) References cited:
- CN-B- 109 009 509
- US-A1- 2023 301 755

## Description

### Technical Field

The disclosure belongs to the technical field of dental implantation, and in particular to a method and apparatus for displaying scanning data, a device, and a storage medium.

### Background

Before dental implantation, it is necessary to scan gum data of a user. In the related art, a rectangular cube scanning rod with a separate code is identified during scanning to determine a spatial position of an implant based on the scanning data. However, in the related art, a scanning process and a current scanning state of an identification point are not perceived by the user.

Patent document US2023301755 discloses a method and apparatus for acquiring digital oral cavity data and a tooth scanner control system. In the method the scanning data of the target scanning rod is matched with standard data of the target scanning rod; and the matched standard data of the target scanning rod is utilized for replacing the scanning data of the target scanning rod in the scanning data, and the digital oral cavity data of the target object is obtained.

Patent document CN109009509 discloses a digital oral cavity model establishing method, involving mounting scanning rod assembly on implant in patient mouth, obtaining three-dimensional data image of oral cavity, and establishing digital oral cavity model.

### Summary

For the above problems, embodiments of the disclosure provide a method and apparatus for displaying scanning data, a device, and a storage medium, which can display a scanning process and a current scanning state of an identification point.

The embodiments of the disclosure provide a method for displaying scanning data, applied to a scanning scene with a scanning rod, and including:
in the scanning scene, identifying identification points of the scanning rod, and determining state information of the various identification points; and
displaying the various identification points based on the state information of the various identification points.

In some embodiments, the state information includes a first state and a second state; and the displaying the various identification points based on the state information of the various identification points includes:
displaying the identification points in the first state based on a first display mode, and displaying the identification points in the second state based on a second display mode, the first display mode and the second display mode are different.

In some embodiments, the determining state information of the various identification points includes:
determining whether the various identification points meet a scanning requirement, determining identification points that meet the scanning requirement as the identification points in the first state, and determining identification points that do not meet the scanning requirement as the identification points in the second state.

In some embodiments, the determining whether the various identification points meet a scanning requirement, determining identification points that meet the scanning requirement as the identification points in the first state, and determining identification points that do not meet the scanning requirement as the identification points in the second state includes:
determining whether numbers of scanning times of the various identification points satisfy a number-of-scanning-times threshold;
determining identification points that satisfy the number-of-scanning-times threshold as the identification points in the first state; and
determining identification points that do not satisfy the number-of-scanning-times threshold as the identification points in the second state.

In some embodiments, the determining whether the various identification points meet a scanning requirement, determining identification points that meet the scanning requirement as the identification points in the first state, and determining identification points that do not meet the scanning requirement as the identification points in the second state includes:
determining whether numbers of scanning times of the various identification points satisfy a number-of-scanning-times threshold;
determining whether identification points, of which the numbers of scanning times reach the number-of-scanning-times threshold, are matched with identification points in a database; and
determining identification points that are successfully matched, of which the numbers of scanning times reach the number-of-scanning-times threshold, as the identification points in the first state; and determining identification points that are unsuccessfully matched, of which the numbers of scanning times reach the number-of-scanning-times threshold, and identification points, of which the numbers of scanning times do not satisfy the number-of-scanning-times threshold, as the identification points in the second state.

In some embodiments, the determining whether the various identification points meet a scanning requirement, determining identification points that meet the scanning requirement as the identification points in the first state, and determining identification points that do not meet the scanning requirement as the identification points in the second state includes:
determining whether numbers of scanning times of the various identification points satisfy a number-of-scanning-times threshold, and determining whether identification points, of which the numbers of scanning times reach the number-of-scanning-times threshold, are matched with identification points in a database, and determining whether the various identification points are located in a scanning box;
determining identification points that are located in the scanning box and are successfully matched, of which the numbers of scanning times reach the number-of-scanning-times threshold, as the identification points in the first state; and
determining identification points that are located outside the scanning box and are unsuccessfully matched, of which the numbers of scanning times do not satisfy the number-of-scanning-times threshold or the numbers of scanning times reach the number-of-scanning-times threshold, as the identification states in the second state.

In some embodiments, the determining state information of the various identification points includes:
determining whether numbers of scanning times of the various identification points satisfy a number-of-scanning-times threshold, and determining whether identification points, of which the numbers of scanning times reach the number-of-scanning-times threshold, are matched with identification points in a database, and determining whether the various identification points are located in a scanning box;
determining identification points that are located in the scanning box and are successfully matched, of which the numbers of scanning times reach the number-of-scanning-times threshold, as the identification points in a first state;
determining identification points that are located outside the scanning box and are successfully matched, of which the numbers of scanning times reach the number-of-scanning-times threshold, as the identification points in a second state; and
determining identification points that are unsuccessfully matched, of which the numbers of scanning times reach the number-of-scanning-times threshold, or identification points, of which the numbers of scanning times do not satisfy the number-of-scanning-times threshold, as the identification points in a third state.

In some embodiments, the displaying the various identification points based on the state information of the various identification points includes:
displaying the identification points in the first state in a first display mode, displaying the identification points in the second state in a second display mode, and displaying the identification points in a third state in a third display mode, where the first display mode, the second display mode, and the third display mode are different from each other; and when the various identification points are displayed, scanning rod models corresponding to the various identification points are not displayed.

In some embodiments, the method further includes:
changing the display mode of a target identification point in a case that the state information of the target identification point is changed.

In some embodiments, the method further includes:
matching the various identification points with identification points of the scanning rods in a standard database; and
displaying the identification points of a successfully matched scanning rod in the database.

In some embodiments, the method includes:
aligning the various identification points with a general-purpose model in a general-purpose database to obtain a target model; and
displaying the target model.

In some embodiments, the general-purpose model includes three-dimensional models of other scanning rods.

The embodiments of the disclosure provide an apparatus for displaying scanning data, applied to a scanning scene with a scanning rod, and including:
an identification component, configured to: in the scanning scene, identify identification points of the scanning rod, and determine state information of the various identification points; and
a first display component, configured to display the various identification points based on the state information of the various identification points.

The embodiments of the disclosure provide an electronic device, including a memory, a processor, and a computer program stored on the memory and runnable on the processor, where the processor, when executing the computer program, implements any one of the above methods.

The embodiments of the disclosure provide a computer-readable storage medium, having a computer program stored thereon. The computer program, when executed by a processor, implements any one of the above methods.

The embodiments of the disclosure provide a computer program product. The computer program product, when run on a electronic device, causes the electronic device to perform any one of the above methods.

According to the method for displaying scanning data provided in the embodiments of the disclosure, in the scanning scene, the identification points of the scanning rod are identified, and the state information of the various identification points are determined; and the various identification points are displayed based on the state information of the various identification points. Therefore, a scanning process and current scanning states of the identification points can be displayed.

### Brief Description of the Drawings

The disclosure will be described in more detail below in accordance with the embodiments and with reference to the accompanying drawings.
FIG. 1 is a schematic flowchart of implementation of a method for displaying scanning data according to an embodiment of the disclosure;
FIG. 2 is a schematic diagram of displaying of identification points according to an embodiment of the disclosure;
FIG. 3 is a schematic flowchart of implementation of a method for displaying scanning data according to an embodiment of the disclosure;
FIG. 4 is a schematic structural diagram of an apparatus for displaying scanning data according to an embodiment of the disclosure; and
FIG. 5 is a schematic structural diagram of constitution of an electronic device according to an embodiment of the disclosure.

In the accompanying drawings, the same parts are marked with the same numerals, and the accompanying drawings are not drawn to an actual scale.

### Detailed Description

To make the objectives, technical solutions, and advantages of the disclosure clearer, the following further describes the disclosure in detail with reference to the accompanying drawings. The described embodiments are not to be considered as a limitation to the disclosure. All other embodiments obtained by a person of ordinary skill in the art without creative efforts shall fall within the protection scope of the disclosure.

In the following description, "some embodiments" are related and describe subsets of all possible embodiments, but it may be understood that "some embodiments" may be the same subset or different subsets of all the possible embodiments, and can be combined with each other without conflict.

If similar descriptions of "first\second\third" appear in this text, the following explanation will be added. In the following description, the term "first\ second\third" is merely used to distinguish similar players and does not represent a specific order of the players. "First\second\third" can be interchanged in a specific order or precedence where permitted, to enable the embodiments of the disclosure described herein to be implemented in a sequence other than that illustrated or described here.

Unless defined otherwise, all technical and scientific terms used herein have the same meaning as commonly understood by those skilled in the art to which the disclosure belongs. Terms used herein are merely intended to describe objectives of the embodiments of the disclosure, but are not intended to limit the disclosure.

The embodiments of the disclosure provide a method for displaying scanning data that can be applied to electronic devices such as a mobile phone, a tablet, a wearable device, an in-vehicle device, an augmented reality (AR)/virtual reality (VR) device, a laptop, an ultra-mobile personal computer (UMPC), a netbook, a personal digital assistant (PDA), and a scanning device. The embodiments of the disclosure do not impose any restrictions on the specific types of the electronic devices. The functions achieved by the method for displaying scanning data provided in the embodiments of the disclosure can be implemented by calling a program code by a processor of an electronic device. The program code can be stored in a computer storage medium.

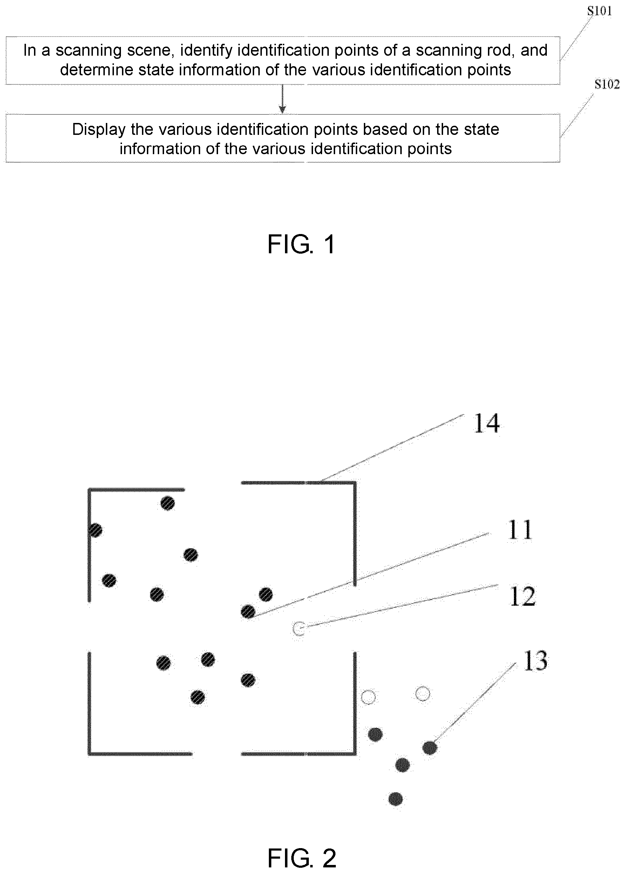

The embodiments of the disclosure provide a method for displaying scanning data. An electronic device used as a scanning device is taken as an example for explanation below. FIG. 1 is a schematic flowchart of implementation of a method for displaying scanning data according to an embodiment of the disclosure. As shown in FIG. 1, the method for displaying scanning data includes the following:
Step S101: in a scanning scene, identification points of a scanning rod are identified, and state information of the various identification points is determined.

In some embodiments of the disclosure, the scanning scene can be: a scene where a scanning rod is mounted on the gum of a user. Scanning the scanning rod on the gum of the user can be achieved by the following step: It first ensures that a scanning device has been calibrated and set. A patient is allowed to sit in a comfortable position. The oral cavity of the patient is ensured to be clean and tidy, without food residues or the like. A mouth opener is fixed at the oral cavity of the patient to ensure that the scanning rod is completely exposed. Then, the scanning rod is placed in the oral cavity of the patient to ensure that the scanning rod is in contact with and closely abuts against the gum. The scanning device is turned on to perform scanning according to a guidance and operation interface of the device.

In some embodiments of the disclosure, the scanning device scans the scanning rod through a specific sensor or camera to identify a position and feature of each identification point. During the scanning of the scanning device, data of scanned points will be acquired in real time. Based on the acquired data, the state information of each identification point will be determined.

In some embodiments of the disclosure, information of the various identification points can be determined, and states of the various identification points can be determined based on the information of the various identification points. The information may include: numbers of scanning times, whether the identification points are inside a scanning box, and whether the identification points are matched with identification points in a database.

In some embodiments of the disclosure, the state information of identification points that meet a scanning requirement can be set as a first state, and the states of identification points that do not meet the scanning requirement can be set as a second state. The first state and the second state are different.

In some embodiments of the disclosure, whether the scanning requirement is met may include the following: whether a number-of-scanning-time threshold is satisfied, whether the identification points are matched with the identification points in the database, and/or whether the identification points are within the scanning box.

Step S102: the various identification points are displayed based on the state information of the various identification points.

In some embodiments of the disclosure, the identification points with different state information can be displayed in different modes.

Exemplarily, the state information may include: a first state and a second state. The identification points in the first state can be displayed based on a first display mode, and the identification points in the second state can be displayed based on a second display mode. The first display mode and the second display mode are different.

In some embodiments, the state information may include: a first state, a second state, and a third state. The identification points in the first state are displayed in the first display mode; the identification points in the second state are displayed in the second display mode; and the identification points in a third state are displayed in a third display mode. The first display mode, the second display mode, and the third display mode are different from each other; and when the various identification points are displayed, scanning rod models corresponding to the various identification points are not displayed.

In some embodiments of the disclosure, different display modes may include different display colors. The various identification points can be displayed on a display screen of the device or a computer connected to the device. When the various identification points are displayed, the various identification points can be displayed in the form of two-dimensional images or three-dimensional models.

According to the method for displaying scanning data provided in the embodiments of the disclosure, in the scanning scene, the identification points of the scanning rod are identified, and the state information of the various identification points are determined; and the various identification points are displayed based on the state information of the various identification points. Therefore, a scanning process and current scanning states of the identification points can be displayed.

In some embodiments, when the various identification points are displayed, the scanning rod models corresponding to the various identification points are not displayed.

In some embodiments of the disclosure, since only identification points are displayed only, the fluency of scanning can be improved.

In some embodiments, the state information includes: a first state and a second state, and step S102 can be implemented through the following steps:
Step S1021: the identification points in the first state are displayed based on the first display mode, and the identification points in the second state are displayed based on the second display mode. The first display mode and the second display mode are different.

In some embodiments of the disclosure, the first display mode and the second display mode can be configured. The two display modes can achieve displaying in different colors or shapes. Exemplarily, displaying the identification points in the first state based on the first display mode may include: displaying the identification points in the first state in green. Displaying the identification points in the second state based on the second display mode may include: displaying the identification points in the second state in yellow. Of course, in other embodiments, displaying the identification points in the first state based on the first display mode may include: displaying the identification points in the first state in blue. Displaying the identification points in the second state based on the second display mode may include: displaying the identification points in the second state in red. Different shapes are taken as an example. Displaying the identification points in the first state based on the first display mode may include: displaying the identification points in the first state in the form of a square box. Displaying identification points in the second state based on the second display mode may include: displaying the identification points in the second state in the form of a circle.

In some embodiments, step S101 may include the following:
Step S1011: it is determined that whether the various identification points meet a scanning requirement; identification points that meet the scanning requirement are determined as the identification points in the first state; and identification points that do not meet the scanning requirement are determined as the identification points in the second state.

In some embodiments of the disclosure, whether the scanning requirement is met may include the following: whether a number-of-scanning-time threshold is satisfied, whether the identification points are matched with the identification points in the database, and/or whether the identification points are within the scanning box, Whether the count of the identification points exceeds the identification points count threshold, and whether the duration of the scan surpasses the time threshold. In some embodiments, this can be achieved by: performing registering operations between identification points and a general-purpose model corresponding to the identification points at preset time intervals or after a preset scanning number; determining whether the identification points in a registered general-purpose models match those in the database; determine identifying matched identification points as identification points in the first state, and determining unmatched I identification points as identification points in the second state.

In some embodiments of the disclosure, each identification point in the scanning rod can be checked to determine whether it meets the scanning requirement. If the identification point meets the scanning requirement, the identification point can be marked as being in the first state. If the identification point does not meet the scanning requirement, the identification point will be marked as being in the second state.

In some embodiments, step S1011 may include the following:
Step S1: whether numbers of scanning times of the various identification points satisfy a number-of-scanning-times threshold is determined.

In some embodiments of the disclosure, the number-of-scanning-times threshold can be configured. Exemplarily, the number-of-scanning-times threshold can be set to 5. In other embodiments, the number-of-scanning-times threshold can be set to 4, 6, or the like.

In some embodiments of the disclosure, in the scanning process of each identification point, the numbers of scanning times of the various identification points can be calculated in real time. After the numbers of scanning times of the various identification points is obtained, the numbers of scanning times of the various identification points and the number-of-scanning-times threshold can be compared to determine whether the numbers of scanning times of the various identification points satisfy the number-of-scanning-times threshold.

Step S2: identification points that do not satisfy the number-of-scanning-times threshold are determined as the identification points in the second state.

In some embodiments of the disclosure, if the number of scanning times of an identification point reaches the number-of-scanning-times threshold, it indicates that the identification point satisfies the number-of-scanning-times threshold, and the identification point that satisfies the number-of-scanning-times threshold is determined as the identification point in the first state.

Continuing from the above example, the number-of-scanning-times threshold being 5 is taken as an example. If the number of scanning times of an identification point is greater than 5, the identification point is an identification point in the first state.

Step S3: identification points that do not satisfy the number-of-scanning-times threshold are determined as the identification points in the second state.

In some embodiments of the disclosure, if the number of scanning times of an identification point is less than the number-of-scanning-times threshold, it indicates that the number of scanning times of the identification point does not satisfy the number-of-scanning-times threshold, and the identification point is an identification point in the second state.

According to the method provided in some embodiments of the disclosure, the identification points in the first state and the identification points in the second state are distinguished solely based on the number-of-scanning-times threshold. By displaying the various identification points, a user can clearly distinguish the state information of different identification points to determine the numbers of scanning times of the identification points.

In some embodiments, step S1011 can be implemented by the following step:
Step S1: whether numbers of scanning times of the various identification points satisfy the number-of-scanning-times threshold is determined.

In some embodiments of the disclosure, the scanning device can record the number of scanning times of each identification point. When the number of scanning times of an identification point reaches the set threshold, it can indicate that the number of scanning times of the identification point meets the requirement.

Step S4: whether identification points, of which the numbers of scanning times reach the number-of-scanning-times threshold, are matched with identification points in a database is determined.

In some embodiments of the disclosure, for identification points, of which the numbers of scanning times reach the threshold, the identification points can be compared and matched with identification point information stored in the database to determine whether the matching is successful.

Step S5: identification points that are successfully matched, of which the numbers of scanning times reach the number-of-scanning-times threshold, are determined as the identification points in the first state; and identification points that are unsuccessfully matched, of which the numbers of scanning times reach the number-of-scanning-times threshold, and identification points, of which the numbers of scanning times do not satisfy the number-of-scanning-times threshold, are determined as the identification points in the second state.

In some embodiments, step S1011 may be implemented through the following step:
Step S6: whether numbers of scanning times of the various identification points satisfy a number-of-scanning-times threshold is determined; and whether identification points, of which the numbers of scanning times reach the number-of-scanning-times threshold, are matched with identification points in a database is determined; and whether the various identification points are located in a scanning box is determined.

In some embodiments of the disclosure, the scanning box is a rectangular box or a boundary used in the scanning device to display a scanning region. This scanning box can help a user accurately locate a scanned region to ensure the accuracy and completeness of scanning. When the scanning device moves on the gum, the scanning box will move too and be displayed in the scanning region. The identification points in the scanning box represent a positional relationship between a scanning window of a scanner and identification points on an upper surface the scanning rod. The user can confirm whether the device covers a region to be scanned by observing the position and size of the scanning box, to obtain comprehensive data information.

Step S7: identification points that are located in the scanning box and are successfully matched, of which the numbers of scanning times reach the number-of-scanning-times threshold, are determined as the identification points in the first state.

Step S8: identification points that are located outside the scanning box and are unsuccessfully matched, of which the numbers of scanning times do not satisfy the number-of-scanning-times threshold or the numbers of scanning times reach the number-of-scanning-times threshold, are determined as the identification states in the second state.

In some embodiments of the disclosure, if an identification point is located in the scanning box, of which the number of scanning times reaches the number-of-scanning-times threshold, or is successfully matched, the state of the identification point is the second state.

In some embodiments, step S101 can be implemented through the following steps:
Step S1012: whether numbers of scanning times of the various identification points satisfy a number-of-scanning-times threshold is determined; and whether identification points, of which the numbers of scanning times reach the number-of-scanning-times threshold, are matched with identification points in a database is determined; and whether the various identification points are located in a scanning box is determined.
Step S1013: identification points that are located in the scanning box and are successfully matched, of which the numbers of scanning times reach the number-of-scanning-times threshold, are determined as the identification points in the first state.
Step S1014: identification points that are located outside the scanning box and are successfully matched, of which the numbers of scanning times reach the number-of-scanning-times threshold, are determined as the identification points in the second state.
Step S1015: identification points that are unsuccessfully matched, of which the numbers of scanning times reach the number-of-scanning-times threshold, or identification points, of which the numbers of scanning times do not satisfy the number-of-scanning-times threshold, are determined as identification points in a third state.

In some embodiments of the disclosure, the identification points can be classified into identification points in three states according to different conditions satisfied, so that a user can learn about a scanning process more easily.

In some embodiments, step S102 can be implemented through the following step:
Step S1022: the identification points in the first state are displayed in a first display mode; the identification points in the second state are displayed in a second display mode; and the identification points in the third state are displayed in a third display mode. The first display mode, the second display mode, and the third display mode are different from each other; and when the various identification points are displayed, scanning rod models corresponding to the various identification points are not displayed.

In some embodiments of the disclosure, the first display mode includes a first-color display mode; the second display mode includes a second-color display mode; and the third display mode includes a third-color display mode. The first color, the second color, and the third color are different from each other. FIG. 2 is a schematic diagram of displaying of identification points according to an embodiment of the disclosure. As shown in FIG. 2, in FIG. 2, 14 represents a scanning box; 11 represents identification points displayed in the first display mode; 12 represents identification points displayed in the second display mode; and 13 represents identification points displayed in the third display mode.

In some embodiments of the disclosure, the first color, the second color, and the third color can be configured. Exemplarily, the first color can be green; the second color can be blue; and the third color can be yellow. In other embodiments, the first color may be light green; the second color may be dark green; and the third color may be yellow.

In some embodiments, the method further comprises
determining whether the various identification points are matched with identification points in a database; calculating a percentage of successfully matched identification points relative to a total number of identification points in the same scanned area (for example, a continuous area with specific features on the gums or tooth surfaces, such as one side of a tooth, a section of the gums, or any plane or curved segment on the scanned object) stored in the database; in a case that the percentage is less than a preset percentage threshold, adding a guiding indicator for the same scanned area; in a case that the guiding indicator indicates that the scanned area requires additional scans, and, displaying the same scanning area in a fourth state, for example, using a color band function to visually alert the user of sections that require greater attention.

According to the method provided in some embodiments of the disclosure, the state information of different identification points can be clearly distinguished.

In some embodiments, the method further includes the following:
The display mode of a target identification point is changed in a case that the state information of the target identification point is changed.

In some embodiments of the disclosure, the states of the various identification points can be determined in real time in the scanning process. If there is a change in the state information of an identification point, the identification point is a target identification point. In this way, the display mode of the target identification point will be changed too. Exemplarily, when the state information of the target identification point is changed from second state information to first state information, the target identification point may change from yellow to light green.

In some embodiments, after step S102, the method further includes the following:
Step S103: the various identification points are matched with identification points of scanning rods in a standard database.

In some embodiments of the disclosure, the scanning rods in the standard database include a scanning rod that has the same shape as the scanning rod in the scanning scene.

In some embodiments of the disclosure, feature information of the identification points can be determined, and the feature information of three-dimensional models of the identification points of the scanning rods in the standard database can be determined. A similarity between the feature information of the identification points and the feature information of the identification points in the database can be calculated using an algorithm (e.g. the nearest neighbor algorithm). According to a similarity calculation result, which scanning rods corresponding to three-dimensional models of the identification points are best matched with the successfully identified identification points can be determined.

Step S104: the identification points of a successfully matched scanning rod in the database are displayed.

In some embodiments of the disclosure, a first target three-dimensional model that is successfully matched is displayed for viewing and analysis by a user, so that the user can learn about a scanning progress and other information.

According to the method provided in some embodiments of the disclosure, the identification points are matched with the three-dimensional models of the identification points corresponding to the various scanning rods in the standard database, so that in a case that a first target three-dimensional model is matched with the successfully identified identification points, the first target three-dimensional model is displayed. In this way, a user can view scanning data more intuitively.

In some embodiments, before step S103, the method further includes the following:
Step S1031: correspondence relationships between the identification points and the three-dimensional models are established.

In the embodiments of the disclosure, a data structure can be defined, and a table or object can be used to represent the three-dimensional model corresponding to each identification point. The table is taken as an example. A table or a set can be established. Each row represents a correspondence relationship, including identifiers of the identification points and corresponding three-dimensional model identifiers.

Step S1032: the correspondence relationships are stored in the database.

According to the method provided in the embodiment of the disclosure, the correspondence relationships between the identification points and the three-dimensional models are established, and these relationships are effectively stored in the database for subsequent matching and query operations.

In some embodiments, after step S104, the method further includes the following:
Step S105: the various identification points are aligned with a general-purpose model in a general-purpose database to obtain a target model.

In some embodiments of the disclosure, the general-purpose model includes three-dimensional models of other scanning rods.

In some embodiments of the disclosure, position information of the scanning rod in the scanning scene can be determined based on the various identification points, and the general-purpose model in the general-purpose database can be determined based on the position information. Then, the various identification points can be aligned with the general-purpose model in the general-purpose database to obtain the target model. The target model includes the general-purpose model and the three-dimensional models of the identification points.

Step S106: the target model is displayed.

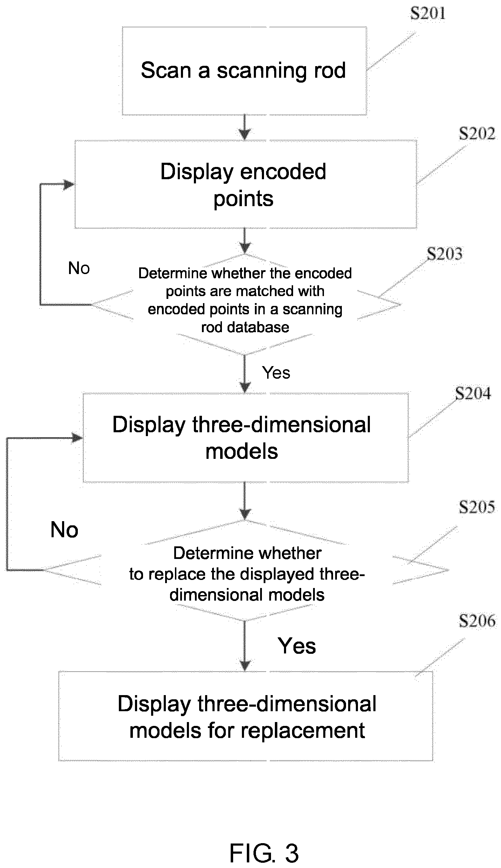

Based on the aforementioned embodiments, the embodiments of the disclosure further provide a method for displaying scanning data. FIG. 3 is a schematic flowchart of implementation of a method for displaying scanning data according to an embodiment of the disclosure. As shown in FIG. 3, the method includes the following:
Step S201: a scanning rod is scanned.

The scanning rod can be scanned by a scanning device. There are identification points on the scanning rod.

Step S202: the identification points are displayed.

In some embodiments of the disclosure, the identification points can be displayed according to state information of the identification points.

In some embodiments of the disclosure, identification points that are located in a scanning box and are successfully matched, of which the numbers of scanning times reach a number-of-scanning-times threshold, are determined as identification points in a first state; identification points that are located outside the scanning box and are successfully matched, of which the numbers of scanning times reach the number-of-scanning-times threshold, are determined as identification points in a second state; and identification points that are unsuccessfully matched, of which the numbers of scanning times reach the number-of-scanning-times threshold, or identification points, of which the numbers of scanning times do not satisfy the number-of-scanning-times threshold, are determined as identification points in a third state. The identification points in the first state are displayed in a first display mode; the identification points in the second state are displayed in a second display mode; and the identification points in the third state are displayed in a third display mode. The first display mode, the second display mode, and the third display mode are different from each other; and when the various identification points are displayed, scanning rod models corresponding to the various identification points are not displayed.

Step S203: whether the identification points are matched with identification points in a scanning rod database is determined.

In some embodiments of the disclosure, if yes, step S204 is executed, and if no, step S202 is continued to be executed.

In some embodiments of the disclosure, the scanning rod database includes correspondence relationships between three-dimensional models and the identification points.

Step S204: the three-dimensional models are displayed.

Step S205: whether to replace the displayed three-dimensional models is determined.

In some embodiments of the disclosure, the three-dimensional models of the identification points can be replaced with three-dimensional models in a general-purpose database. The three-dimensional models in the general-purpose database may include three-dimensional models of other scanning rods. The three-dimensional models of other scanning rods are distinguished from three-dimensional models of other scanning rods that are different from the scanning rod in the scanning scene, and are generally three-dimensional models of general-purpose scanning rods commonly used in the industry. In addition, the three-dimensional models in the general-purpose database can also be three-dimensional models of a dental crown, a rod clip, and/or a bridge frame.

In some embodiments of the disclosure, if yes, step S206 is executed. If not, step S204 is executed.

Step S206: three-dimensional models for replacement are displayed.

According to the method provided in some embodiments of the disclosure, only the identification points are displayed when an encoded scanning rod is scanned, and identification states of current points are represented in different colors. It can ensure the scanning fluency. When the identification points are matched with the identification points in the database, the three-dimensional models are displayed, so that a user can more intuitively view comparison between scanning data and actual data in the mouth of a patient. After the matching of all the encoded scanning rods is completed, the three-dimensional models can be transformed into other general-purpose scanning rod models for subsequent prosthesis design (such as a dental crown, a rod clip, and a bridge frame).

According to the aforementioned embodiments, the embodiments of the disclosure provide an apparatus for displaying scanning data. The apparatus includes various components and units included in the various component, and can be implemented by a processor in a computer device. Of course, the apparatus can also be implemented through a specific logic circuit. During implementation, the processor can be a central processing unit (CPU), a microprocessor unit (MPU), a digital signal processing (DSP), a field programmable gate array (FPGA), and the like.

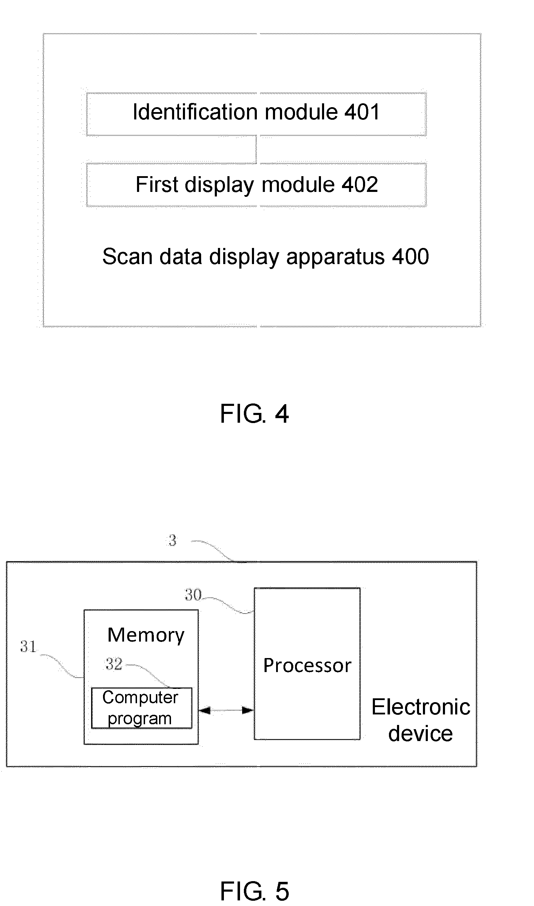

The embodiments of the disclosure provide an apparatus for displaying scanning data. FIG. 4 is a schematic structural diagram of an apparatus for displaying scanning data according to an embodiment of the disclosure. As shown in FIG. 4, the apparatus for displaying scanning data 400 includes:
an identification component 401, configured to: in the scanning scene, identify identification points of the scanning rod, and determine state information of the various identification points; and
a first display component 402, configured to display the various identification points based on the state information of the various identification points.

In some embodiments, the state information includes a first state and a second state. The first display component includes:
a first display unit, configured to: display the identification points in the first state based on a first display mode, and display the identification points in the second state based on a second display mode. The first display mode and the second display mode are different.

In some embodiments, the identification component includes:
a first identification unit, configured to: determine whether the various identification points meet a scanning requirement, determine identification points that meet the scanning requirement as the identification points in the first state, and determine identification points that do not meet the scanning requirement as the identification points in the second state.

In some embodiments, the first identification unit includes:
a first determination subunit, configured to determine whether numbers of scanning times of the various identification points satisfy a number-of-scanning-times threshold;
a second determination subunit, configured to: determine identification points that satisfy the number-of-scanning-times threshold as the identification points in the first state; and
a third determination subunit, configured to: determine identification points that do not satisfy the number-of-scanning-times threshold as the identification points in the second state.

In some embodiments, the first identification unit includes:
a fourth determination subunit, configured to determine whether numbers of scanning times of the various identification points satisfy a number-of-scanning-times threshold;
a fifth determination subunit, configured to: determine whether identification points, of which the numbers of scanning times reach the number-of-scanning-times threshold, are matched with identification points in a database; and a sixth determination subunit, configured to: determine identification points that are successfully matched, of which the numbers of scanning times reach the number-of-scanning-times threshold, as the identification points in the first state; and determine identification points that are unsuccessfully matched, of which the numbers of scanning times reach the number-of-scanning-times threshold, and identification points, of which the numbers of scanning times do not satisfy the number-of-scanning-times threshold, as the identification points in the second state.

In some embodiments, the first identification unit includes: a seventh determination subunit, configured to: determine whether numbers of scanning times of the various identification points satisfy a number-of-scanning-times threshold, and determine whether identification points, of which the numbers of scanning times reach the number-of-scanning-times threshold, are matched with identification points in a database, and determine whether the various identification points are located in a scanning box; an eighth determination subunit, configured to determine identification points that are located in the scanning box and are successfully matched, of which the numbers of scanning times reach the number-of-scanning-times threshold, as the identification points in the first state; and a ninth determination subunit, configured to determine identification points that are located outside the scanning box and are unsuccessfully matched, of which the numbers of scanning times do not satisfy the number-of-scanning-times threshold or the numbers of scanning times reach the number-of-scanning-times threshold, as the identification states in the second state.

In some embodiments, the identification component includes: a first determination unit, configured to: determine whether numbers of scanning times of the various identification points satisfy a number-of-scanning-times threshold, and determine whether identification points, of which the numbers of scanning times reach the number-of-scanning-times threshold, are matched with identification points in a database, and determine whether the various identification points are located in the scanning box; a second determination unit, configured to determine identification points that are located in the scanning box and are successfully matched, of which the numbers of scanning times reach the number-of-scanning-times threshold, as the identification points in the first state; a third determination unit, configured to determine identification points that are located outside the scanning box and are successfully matched, of which the numbers of scanning times reach the number-of-scanning-times threshold, as the identification points in the second state; and a fourth determination unit, configured to determine identification points that are unsuccessfully matched, of which the numbers of scanning times reach the number-of-scanning-times threshold, or identification points, of which the numbers of scanning times do not satisfy the number-of-scanning-times threshold, as identification points in a third state.

The first display component is configured to display the various identification points based on the state information of the various identification points, including:
a second display unit, configured to: display the identification points in the first state in a first display mode, display the identification points in the second state in a second display mode, and display the identification points in the third state in a third display mode. The first display mode, the second display mode, and the third display mode are different from each other; and when the various identification points are displayed, scanning rod models corresponding to the various identification points are not displayed.

In some embodiments, the scanning data display apparatus includes: a change component, configured to change the display mode of a target identification point in a case that the state information of the target identification point is changed.

In some embodiments, the scanning data display apparatus includes: a matching component, configured to match the various identification points with identification points of scanning rods in a standard database; and a second display component, configured to display the identification points of a successfully matched scanning rod in the database.

In some embodiments, the scanning data display apparatus includes: a aligning component, configured to align the various identification points with a general-purpose model in a general-purpose database to obtain a target model; and a third display component, configured to display the target model.

In some embodiments, the general-purpose model includes three-dimensional models of other scanning rods.

A person skilled in the art can clearly know that for ease and simplicity of description, division of all the above functional units and components is exemplified only. In practical applications, the foregoing functions may be allocated to be completed by different functional units and components as required, that is, an inner structure of the apparatus is divided into different functional units and components, so as to complete all or some of the functions described above. The functional units and components in the embodiments may be all integrated into one processing unit, may also physically exist separately, or may be integrated into one unit by two or more units. The above integrated unit can be implemented in the form of hardware or in the form of software functional units. In addition, the specific names of the functional units and components are only for the convenience of distinguishing each other, and are not intended to limit the scope of protection of this application. For specific work processes of the units and components in the above system, reference may be made to the corresponding processes in the foregoing method embodiments. Details are not described herein again.

FIG. 5 is a schematic structural diagram of an electronic device according to an embodiment of the disclosure. As shown in FIG. 5, the electronic device 3 of this embodiment may include: at least one processor 30 (only one processor 30 is shown in FIG. 5), a memory 31, and a computer program 32 stored on the memory 31 and runnable on the at least one processor 30. When executing the computer program 32, the processor 30 implements the steps in any of the above method embodiments, such as step S101 to step S102 in the embodiment shown in FIG. 1. Or, when executing the computer program 32, the processor 30 implements the functions of the various components/units in the foregoing apparatus embodiments, such as the functions of the component 401 to the component 402 shown in FIG. 4.

Exemplarily, the computer program 32 can be divided into one or more components/units. The one or more components/units are stored in the memory 31 and executed by the processor 30 to complete the disclosure. The one or more components/units can be a series of instruction segments of the computer program 32 capable of completing specific functions. The instruction segments are used for describing an execution process of the computer program 32 in the electronic device 3.

The embodiments of the disclosure further provide a computer-readable storage medium, having a computer program 32 stored thereon. The computer program 32, when executed by a processor 30, can implement the steps in the above method embodiments.

The embodiments of the disclosure provide a computer program product. The computer program product, when run, can implement the steps in the above method embodiments.

When the integrated unit is implemented in the form of a software functional unit and sold or used as an independent product, the integrated unit may be stored in a computer-readable storage medium. Based on this understanding, the disclosure can implement all or part of the processes in the methods of the above embodiments by instructing relevant hardware through computer program 32. The computer program 32 can be stored in a computer-readable storage medium. When executed by a processor 30, the computer program 32 can implement the steps of the above method embodiments. The computer program 32 includes computer program code. The computer program code may be in a source code form, an object code form, an executable file form, or some intermediate forms, etc. The computer-readable medium may at least include any entity or apparatus capable of carrying computer program codes to a terminal, a recording medium, a computer memory, a Read-Only Memory (ROM), a Random Access Memory (RAM), an electric carrier signal, a telecommunication signal, and a software distribution medium, such as a USB flash disk, a portable hard disk drive, a magnetic disk, or a compact disc. In some jurisdictions, according to the legislation and patent practice, the computer-readable medium may not be an electric carrier signal and a telecommunication signal.

In the foregoing embodiments, the description of each embodiment has a respective focus. For a part that is not described in detail or recorded in an embodiment, reference may be made to related descriptions in other embodiments.

A person of ordinary skill in the art may notice that the exemplary units and algorithm steps described with reference to the embodiments disclosed in the present specification can be implemented in electronic hardware, or a combination of computer software and electronic hardware. Whether the functions are executed in hardware or software depends on particular applications and design constraint conditions of the technical solutions. A person skilled in the art may implement the described functions by different methods for each particular application, but such implementation is not to be considered beyond the scope of this application.

In the embodiments provided in this application, it should be understood that the disclosed apparatus/network device and method may be implemented in other manners. For example, the described apparatus/network device embodiment is merely an example. For example, the component or unit division is merely logical function division and may be other division during actual implementation. For example, multiple units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electrical, mechanical, or other forms.

The units described as separate components may or may not be physically separated, and the components displayed as units may or may not be physical units, that is, may be located in one place or may be distributed over a plurality of network units. Some or all of the units may be selected according to actual needs to achieve the objectives of the solutions of the embodiments.

The embodiments described above are only used to explain the technical solutions of the disclosure, and are not limited thereto. Although the disclosure has been described in detail with reference to the foregoing embodiments, those of ordinary skill in the art should understand that the technical solutions described in the foregoing embodiments may still be modified, or some of the technical features may be replaced. Such modifications or replacements do not cause the essence of corresponding technical solutions to depart from the spirit and scope of the technical solutions of the respective embodiments of the disclosure, and are intended to be included within the scope of the disclosure.

## Claims

1. A method for displaying scanning data, applied to a scanning scene with a scanning rod, and **characterized by** comprising:
in the scanning scene, identifying identification points of the scanning rod, and determining state information of various identification points; and
displaying the various identification points based on the state information of the various identification points.

2. The method as claimed in claim 1, wherein the state information comprises a first state and a second state; and the displaying the various identification points based on the state information of the various identification points comprises:
displaying the identification points in the first state based on a first display mode, and displaying the identification points in the second state based on a second display mode, the first display mode and the second display mode are different.

3. The method as claimed in claim 2, wherein the determining state information of the various identification points comprises:
determining whether the various identification points meet a scanning requirement, determining identification points that meet the scanning requirement as the identification points in the first state, and determining identification points that do not meet the scanning requirement as the identification points in the second state.

4. The method as claimed in claim 3, wherein the determining whether the various identification points meet the scanning requirement, determining identification points that meet the scanning requirement as the identification points in the first state, and determining identification points that do not meet the scanning requirement as the identification points in the second state comprises:
determining whether numbers of scanning times of the various identification points satisfy a number-of-scanning-times threshold;
determining identification points that satisfy the number-of-scanning-times threshold as the identification points in the first state; and
determining identification points that do not satisfy the number-of-scanning-times threshold as the identification points in the second state.

5. The method as claimed in claim 3 or 4, wherein the determining whether the various identification points meet the scanning requirement, determining identification points that meet the scanning requirement as the identification points in the first state, and determining identification points that do not meet the scanning requirement as the identification points in the second state comprises:
determining whether numbers of scanning times of the various identification points satisfy a number-of-scanning-times threshold;
determining whether identification points, of which the numbers of scanning times reach the number-of-scanning-times threshold, are matched with identification points in a database; and
determining identification points that are successfully matched, of which the numbers of scanning times reach the number-of-scanning-times threshold, as the identification points in the first state; and determining identification points that are unsuccessfully matched, of which the numbers of scanning times reach the number-of-scanning-times threshold, and identification points, of which the numbers of scanning times do not satisfy the number-of-scanning-times threshold, as the identification points in the second state.

6. The method as claimed in any one of claims 3 to 5, wherein the determining whether the various identification points meet the scanning requirement, determining identification points that meet the scanning requirement as the identification points in the first state, and determining identification points that do not meet the scanning requirement as the identification points in the second state comprises:
determining whether numbers of scanning times of the various identification points satisfy a number-of-scanning-times threshold, and determining whether identification points, of which the numbers of scanning times reach the number-of-scanning-times threshold, are matched with identification points in a database, and determining whether the various identification points are located in a scanning box;
determining identification points that are located in the scanning box and are successfully matched, of which the numbers of scanning times reach the number-of-scanning-times threshold, as the identification points in the first state; and
determining identification points that are located outside the scanning box and are unsuccessfully matched, of which the numbers of scanning times do not satisfy the number-of-scanning-times threshold or the numbers of scanning times reach the number-of-scanning-times threshold, as the identification states in the second state.

7. The method as claimed in any one of claims 3 to 6, wherein the determining whether the various identification points meet the scanning requirement, determining identification points that meet the scanning requirement as the identification points in the first state, and determining identification points that do not meet the scanning requirement as the identification points in the second state comprises:
determining whether the various identification points are matched with identification points in a database;
determining identification points that are successfully matched as the identification points in the first state; and
determining identification points that are unsuccessfully matched as the identification states in the second state.

8. The method as claimed in any one of claims 3 to 7, wherein the determining whether the various identification points meet the scanning requirement, determining identification points that meet the scanning requirement as the identification points in the first state, and determining identification points that do not meet the scanning requirement as the identification points in the second state comprises:
determining whether the various identification points are located in a scanning box;
determining identification points that are located in the scanning box as the identification points in the first state; and
determining identification points that are located outside the scanning box as the identification states in the second state.

9. The method as claimed in any one of claims 3 to 8, wherein the determining whether the various identification points meet the scanning requirement, determining identification points that meet the scanning requirement as the identification points in the first state, and determining identification points that do not meet the scanning requirement as the identification points in the second state comprises:
performing registering operations between the various identification points and a general-purpose model corresponding to the various identification points at preset time intervals or after a preset scanning number;
determining whether the various identification points in a registered general-purpose models match identification points in the database;
determine matched identification points as identification points in the first state, and determining unmatched identification points as identification points in the second state.

10. The method as claimed in any one of the preceding claims, wherein the determining state information of the various identification points comprises:
determining whether numbers of scanning times of the various identification points satisfy a number-of-scanning-times threshold, and determining whether identification points, of which the numbers of scanning times reach the number-of-scanning-times threshold, are matched with identification points in a database, and determining whether the various identification points are located in a scanning box; and
determining identification points that are located in the scanning box and are successfully matched, of which the numbers of scanning times reach the number-of-scanning-times threshold, as the identification points in a first state;
determining identification points that are located outside the scanning box and are successfully matched, of which the numbers of scanning times reach the number-of-scanning-times threshold, as the identification points in a second state; and
determining identification points that are unsuccessfully matched, of which the numbers of scanning times reach the number-of-scanning-times threshold, or identification points, of which the numbers of scanning times do not satisfy the number-of-scanning-times threshold, as the identification points in a third state.

11. The method as claimed in claim 10, wherein the displaying the various identification points based on the state information of the various identification points comprises:
displaying the identification points in the first state in a first display mode, displaying the identification points in the second state in a second display mode, and displaying the identification points in a third state in a third display mode, wherein the first display mode, the second display mode, and the third display mode are different from each other; and when the various identification points are displayed, scanning rod models corresponding to the various identification points are not displayed.

12. The method as claimed in any one of claims 3 to 11, wherein the method further comprises:
determining whether the various identification points are matched with identification points in a database;
calculating a percentage of successfully matched identification points relative to a total number of identification points in the same scanned area stored in the database;
in a case that the percentage is less than a preset percentage threshold, adding a guiding indicator for the same scanned area; in a case that the guiding indicator indicates that the scanned area requires additional scans, and, displaying the same scanning area in a fourth state.

13. The method as claimed in any one of the preceding claims, wherein the method further comprises:
matching the various identification points with identification points of the scanning rods in a standard database; and
displaying the identification points of a successfully matched scanning rod in the standard database.

14. The method as claimed in any one of the preceding claims, wherein the method comprises:
aligning the various identification points with a general-purpose model in a general-purpose database to obtain a target model; and displaying the target model, wherein the general-purpose model comprises three-dimensional models of other scanning rods.

15. An apparatus for displaying scanning data, applied to a scanning scene with a scanning rod, **characterized by** comprising:
an identification component, configured to: in the scanning scene, identify identification points of the scanning rod, and determine state information of various identification points; and a first display component, configured to display the various identification points based on the state information of the various identification points.

## Patentansprüche

1. Verfahren zum Anzeigen von Scandaten, angewendet auf eine Scan-Szene mit einem Scan-Stab, **dadurch gekennzeichnet, dass** es Folgendes umfasst:
in der Scan-Szene Identifikationspunkte des Scan-Stabs identifizieren und Zustandsinformationen der verschiedenen Identifikationspunkte bestimmen; und
die verschiedenen Identifikationspunkte basierend auf den Zustandsinformationen der verschiedenen Identifikationspunkte anzeigen.

2. Verfahren nach Anspruch 1, wobei die Zustandsinformationen einen ersten Zustand und einen zweiten Zustand umfassen; und das Anzeigen der verschiedenen Identifikationspunkte basierend auf den Zustandsinformationen der verschiedenen Identifikationspunkte Folgendes umfasst:
Anzeigen der Identifikationspunkte im ersten Zustand in einem ersten Anzeigemodus und Anzeigen der Identifikationspunkte im zweiten Zustand in einem zweiten Anzeigemodus, wobei sich der erste Anzeigemodus und der zweite Anzeigemodus unterscheiden.

3. Verfahren nach Anspruch 2, wobei das Bestimmen der Zustandsinformationen der verschiedenen Identifikationspunkte Folgendes umfasst:
Bestimmen, ob die verschiedenen Identifikationspunkte eine Scan-Anforderung erfüllen, Bestimmen der Identifikationspunkte, die die Scan-Anforderung erfüllen, als die Identifikationspunkte im ersten Zustand, und Bestimmen der Identifikationspunkte, die die Scan-Anforderung nicht erfüllen, als die Identifikationspunkte im zweiten Zustand.

4. Verfahren nach Anspruch 3, wobei das Bestimmen, ob die verschiedenen Identifikationspunkte die Scan-Anforderung erfüllen, das Bestimmen der Identifikationspunkte, die die Scan-Anforderung erfüllen, als die Identifikationspunkte im ersten Zustand, und das Bestimmen der Identifikationspunkte, die die Scan-Anforderung nicht erfüllen, als die Identifikationspunkte im zweiten Zustand Folgendes umfasst:
Bestimmen, ob die Anzahl der Scan-Vorgänge der verschiedenen Identifikationspunkte einen Scan-Anzahl-Schwellwert erfüllt;
Bestimmen der Identifikationspunkte, die den Scan-Anzahl-Schwellwert erfüllen, als die Identifikationspunkte im ersten Zustand; und
Bestimmen der Identifikationspunkte, die den Scan-Anzahl-Schwellwert nicht erfüllen, als die Identifikationspunkte im zweiten Zustand.

5. Verfahren nach Anspruch 3 oder 4, wobei das Bestimmen, ob die verschiedenen Identifikationspunkte die Scan-Anforderung erfüllen, das Bestimmen der Identifikationspunkte, die die Scan-Anforderung erfüllen, als die Identifikationspunkte im ersten Zustand, und das Bestimmen der Identifikationspunkte, die die Scan-Anforderung nicht erfüllen, als die Identifikationspunkte im zweiten Zustand Folgendes umfasst:
Bestimmen, ob die Anzahl der Scan-Vorgänge der verschiedenen Identifikationspunkte einen Scan-Anzahl-Schwellwert erfüllt;
Bestimmen, ob die Identifikationspunkte, deren Anzahl der Scan-Vorgänge den Scan-Anzahl-Schwellwert erreicht, mit Identifikationspunkten in einer Datenbank übereinstimmen; und
Bestimmen der erfolgreich übereinstimmenden Identifikationspunkte, deren Anzahl der Scan-Vorgänge den Scan-Anzahl-Schwellwert erreicht, als die Identifikationspunkte im ersten Zustand; und Bestimmen der nicht erfolgreich übereinstimmenden Identifikationspunkte, deren Anzahl der Scan-Vorgänge den Scan-Anzahl-Schwellwert erreicht, und der Identifikationspunkte, deren Anzahl der Scan-Vorgänge den Scan-Anzahl-Schwellwert nicht erfüllt, als die Identifikationspunkte im zweiten Zustand.

6. Verfahren nach einem der Ansprüche 3 bis 5, wobei das Bestimmen, ob die verschiedenen Identifikationspunkte die Scan-Anforderung erfüllen, das Bestimmen der Identifikationspunkte, die die Scan-Anforderung erfüllen, als die Identifikationspunkte im ersten Zustand, und das Bestimmen der Identifikationspunkte, die die Scan-Anforderung nicht erfüllen, als die Identifikationspunkte im zweiten Zustand Folgendes umfasst:
Bestimmen, ob die Anzahl der Scan-Vorgänge der verschiedenen Identifikationspunkte einen Scan-Anzahl-Schwellwert erfüllt, und Bestimmen, ob die Identifikationspunkte, deren Anzahl der Scan-Vorgänge den Scan-Anzahl-Schwellwert erreicht, mit Identifikationspunkten in einer Datenbank übereinstimmen, und Bestimmen, ob die verschiedenen Identifikationspunkte sich in einem Scan-Bereich befinden;
Bestimmen der Identifikationspunkte, die sich im Scan-Bereich befinden und erfolgreich übereinstimmen, deren Anzahl der Scan-Vorgänge den Scan-Anzahl-Schwellwert erreicht, als die Identifikationspunkte im ersten Zustand; und
Bestimmen der Identifikationspunkte, die sich außerhalb des Scan-Bereichs befinden und nicht erfolgreich übereinstimmen, deren Anzahl der Scan-Vorgänge den Scan-Anzahl-Schwellwert nicht erfüllt oder deren Anzahl der Scan-Vorgänge den Scan-Anzahl-Schwellwert erreicht, als die Identifikationszustände im zweiten Zustand.

7. Verfahren nach einem der Ansprüche 3 bis 6, wobei das Bestimmen, ob die verschiedenen Identifikationspunkte die Scan-Anforderung erfüllen, das Bestimmen der Identifikationspunkte, die die Scan-Anforderung erfüllen, als die Identifikationspunkte im ersten Zustand, und das Bestimmen der Identifikationspunkte, die die Scan-Anforderung nicht erfüllen, als die Identifikationspunkte im zweiten Zustand Folgendes umfasst:
Bestimmen, ob die verschiedenen Identifikationspunkte mit Identifikationspunkten in einer Datenbank übereinstimmen;
Bestimmen der erfolgreich übereinstimmenden Identifikationspunkte als die Identifikationspunkte im ersten Zustand; und
Bestimmen der nicht erfolgreich übereinstimmenden Identifikationspunkte als die Identifikationszustände im zweiten Zustand.

8. Verfahren nach einem der Ansprüche 3 bis 7, wobei das Bestimmen, ob die verschiedenen Identifikationspunkte die Scan-Anforderung erfüllen, das Bestimmen der Identifikationspunkte, die die Scan-Anforderung erfüllen, als die Identifikationspunkte im ersten Zustand, und das Bestimmen der Identifikationspunkte, die die Scan-Anforderung nicht erfüllen, als die Identifikationspunkte im zweiten Zustand Folgendes umfasst:
Bestimmen, ob die verschiedenen Identifikationspunkte sich in einem Scan-Bereich befinden;
Bestimmen der Identifikationspunkte, die sich im Scan-Bereich befinden, als die Identifikationspunkte im ersten Zustand; und
Bestimmen der Identifikationspunkte, die sich außerhalb des Scan-Bereichs befinden, als die Identifikationszustände im zweiten Zustand.

9. Verfahren nach einem der Ansprüche 3 bis 8, wobei das Bestimmen, ob die verschiedenen Identifikationspunkte die Scan-Anforderung erfüllen, das Bestimmen der Identifikationspunkte, die die Scan-Anforderung erfüllen, als die Identifikationspunkte im ersten Zustand, und das Bestimmen der Identifikationspunkte, die die Scan-Anforderung nicht erfüllen, als die Identifikationspunkte im zweiten Zustand Folgendes umfasst:
Durchführen von Registrierungsvorgängen zwischen den verschiedenen Identifikationspunkten und einem allgemeinen Modell, das den verschiedenen Identifikationspunkten entspricht, in vorgegebenen Zeitintervallen oder nach einer vorgegebenen Scan-Anzahl;
Bestimmen, ob die verschiedenen Identifikationspunkte in einem registrierten allgemeinen Modell mit Identifikationspunkten in der Datenbank übereinstimmen;
Bestimmen der übereinstimmenden Identifikationspunkte als Identifikationspunkte im ersten Zustand und Bestimmen der nicht übereinstimmenden Identifikationspunkte als Identifikationspunkte im zweiten Zustand.

10. Verfahren nach einem der vorhergehenden Ansprüche, wobei das Bestimmen der Zustandsinformationen der verschiedenen Identifikationspunkte Folgendes umfasst: Bestimmen, ob die Anzahl der Scan-Vorgänge der verschiedenen Identifikationspunkte einen Scan-Anzahl-Schwellwert erfüllt, und Bestimmen, ob die Identifikationspunkte, deren Anzahl der Scan-Vorgänge den Scan-Anzahl-Schwellwert erreicht, mit Identifikationspunkten in einer Datenbank übereinstimmen, und Bestimmen, ob die verschiedenen Identifikationspunkte sich in einem Scan-Bereich befinden; und
Bestimmen der Identifikationspunkte, die sich im Scan-Bereich befinden und erfolgreich übereinstimmen, deren Anzahl der Scan-Vorgänge den Scan-Anzahl-Schwellwert erreicht, als die Identifikationspunkte in einem ersten Zustand;
Bestimmen der Identifikationspunkte, die sich außerhalb des Scan-Bereichs befinden und erfolgreich übereinstimmen, deren Anzahl der Scan-Vorgänge den Scan-Anzahl-Schwellwert erreicht, als die Identifikationspunkte in einem zweiten Zustand; und
Bestimmen der Identifikationspunkte, die nicht erfolgreich übereinstimmen, deren Anzahl der Scan-Vorgänge den Scan-Anzahl-Schwellwert erreicht, oder der Identifikationspunkte, deren Anzahl der Scan-Vorgänge den Scan-Anzahl-Schwellwert nicht erfüllt, als die Identifikationspunkte in einem dritten Zustand.

11. Verfahren nach Anspruch 10, wobei das Anzeigen der verschiedenen Identifikationspunkte basierend auf den Zustandsinformationen der verschiedenen Identifikationspunkte Folgendes umfasst:
Anzeigen der Identifikationspunkte im ersten Zustand in einem ersten Anzeigemodus, Anzeigen der Identifikationspunkte im zweiten Zustand in einem zweiten Anzeigemodus und Anzeigen der Identifikationspunkte in einem dritten Zustand in einem dritten Anzeigemodus, wobei sich der erste Anzeigemodus, der zweite Anzeigemodus und der dritte Anzeigemodus voneinander unterscheiden; und wenn die verschiedenen Identifikationspunkte angezeigt werden, werden Scan-Stab-Modelle, die den verschiedenen Identifikationspunkten entsprechen, nicht angezeigt.

12. Verfahren nach einem der Ansprüche 3 bis 11, wobei das Verfahren ferner Folgendes umfasst:
Bestimmen, ob die verschiedenen Identifikationspunkte mit Identifikationspunkten in einer Datenbank übereinstimmen;
Berechnen eines Prozentsatzes der erfolgreich übereinstimmenden Identifikationspunkte im Verhältnis zu einer Gesamtzahl von Identifikationspunkten im selben gescannten Bereich, die in der Datenbank gespeichert sind;
in einem Fall, in dem der Prozentsatz unter einem vorgegebenen Prozentsatz-Schwellwert liegt, Hinzufügen eines Führungsindikators für denselben gescannten Bereich; in einem Fall, in dem der Führungsindikator anzeigt, dass der gescannte Bereich zusätzliche Scans erfordert, Anzeigen desselben Scan-Bereichs in einem vierten Zustand.

13. Verfahren nach einem der vorhergehenden Ansprüche, wobei das Verfahren ferner Folgendes umfasst:
Abgleichen der verschiedenen Identifikationspunkte mit Identifikationspunkten der Scan-Stäbe in einer Standarddatenbank; und
Anzeigen der Identifikationspunkte eines erfolgreich abgeglichenen Scan-Stabs in der Standarddatenbank.

14. Verfahren nach einem der vorhergehenden Ansprüche, wobei das Verfahren Folgendes umfasst:
Ausrichten der verschiedenen Identifikationspunkte mit einem allgemeinen Modell in einer allgemeinen Datenbank, um ein Zielmodell zu erhalten; und Anzeigen des Zielmodells, wobei das allgemeine Modell dreidimensionale Modelle anderer Scan-Stäbe umfasst.

15. Vorrichtung zum Anzeigen von Scandaten, angewendet auf eine Scan-Szene mit einem Scan-Stab, **dadurch gekennzeichnet, dass** sie Folgendes umfasst:
eine Identifikationskomponente, die konfiguriert ist, um: in der Scan-Szene Identifikationspunkte des Scan-Stabs zu identifizieren und Zustandsinformationen der verschiedenen Identifikationspunkte zu bestimmen; und eine erste Anzeigekomponente, die konfiguriert ist, um die verschiedenen Identifikationspunkte basierend auf den Zustandsinformationen der verschiedenen Identifikationspunkte anzuzeigen.

## Revendications

1. Procédé d'affichage de données de balayage, appliqué à une scène de balayage avec une tige de balayage, **caractérisé en ce qu'**il comprend :
dans la scène de balayage, identifier des points d'identification de la tige de balayage et déterminer des informations d'état des différents points d'identification ; et
afficher les différents points d'identification en fonction des informations d'état des différents points d'identification.

2. Procédé selon la revendication 1, dans lequel les informations d'état comprennent un premier état et un deuxième état ; et l'affichage des différents points d'identification en fonction des informations d'état des différents points d'identification comprend :
afficher les points d'identification dans le premier état selon un premier mode d'affichage, et afficher les points d'identification dans le deuxième état selon un deuxième mode d'affichage, le premier mode d'affichage et le deuxième mode d'affichage étant différents.

3. Procédé selon la revendication 2, dans lequel la détermination des informations d'état des différents points d'identification comprend :
déterminer si les différents points d'identification satisfont une exigence de balayage, déterminer les points d'identification qui satisfont l'exigence de balayage comme les points d'identification dans le premier état, et déterminer les points d'identification qui ne satisfont pas l'exigence de balayage comme les points d'identification dans le deuxième état.

4. Procédé selon la revendication 3, dans lequel la détermination si les différents points d'identification satisfont l'exigence de balayage, la détermination des points d'identification qui satisfont l'exigence de balayage comme les points d'identification dans le premier état, et la détermination des points d'identification qui ne satisfont pas l'exigence de balayage comme les points d'identification dans le deuxième état comprend :
déterminer si les nombres de fois de balayage des différents points d'identification satisfont un seuil de nombre de fois de balayage ;
déterminer les points d'identification qui satisfont le seuil de nombre de fois de balayage comme les points d'identification dans le premier état ; et
déterminer les points d'identification qui ne satisfont pas le seuil de nombre de fois de balayage comme les points d'identification dans le deuxième état.

5. Procédé selon la revendication 3 ou 4, dans lequel la détermination si les différents points d'identification satisfont l'exigence de balayage, la détermination des points d'identification qui satisfont l'exigence de balayage comme les points d'identification dans le premier état, et la détermination des points d'identification qui ne satisfont pas l'exigence de balayage comme les points d'identification dans le deuxième état comprend :
déterminer si les nombres de fois de balayage des différents points d'identification satisfont un seuil de nombre de fois de balayage ;
déterminer si les points d'identification, dont les nombres de fois de balayage atteignent le seuil de nombre de fois de balayage, correspondent à des points d'identification dans une base de données ; et
déterminer les points d'identification qui sont appariés avec succès, dont les nombres de fois de balayage atteignent le seuil de nombre de fois de balayage, comme les points d'identification dans le premier état ; et déterminer les points d'identification qui ne sont pas appariés avec succès, dont les nombres de fois de balayage atteignent le seuil de nombre de fois de balayage, et les points d'identification, dont les nombres de fois de balayage ne satisfont pas le seuil de nombre de fois de balayage, comme les points d'identification dans le deuxième état.

6. Procédé selon l'une quelconque des revendications 3 à 5, dans lequel la détermination si les différents points d'identification satisfont l'exigence de balayage, la détermination des points d'identification qui satisfont l'exigence de balayage comme les points d'identification dans le premier état, et la détermination des points d'identification qui ne satisfont pas l'exigence de balayage comme les points d'identification dans le deuxième état comprend :
déterminer si les nombres de fois de balayage des différents points d'identification satisfont un seuil de nombre de fois de balayage, et déterminer si les points d'identification, dont les nombres de fois de balayage atteignent le seuil de nombre de fois de balayage, correspondent à des points d'identification dans une base de données, et déterminer si les différents points d'identification sont situés dans une boîte de balayage ;
déterminer les points d'identification qui sont situés dans la boîte de balayage et qui sont appariés avec succès, dont les nombres de fois de balayage atteignent le seuil de nombre de fois de balayage, comme les points d'identification dans le premier état ; et
déterminer les points d'identification qui sont situés en dehors de la boîte de balayage et qui ne sont pas appariés avec succès, dont les nombres de fois de balayage ne satisfont pas le seuil de nombre de fois de balayage ou dont les nombres de fois de balayage atteignent le seuil de nombre de fois de balayage, comme les états d'identification dans le deuxième état.

7. Procédé selon l'une quelconque des revendications 3 à 6, dans lequel la détermination si les différents points d'identification satisfont l'exigence de balayage, la détermination des points d'identification qui satisfont l'exigence de balayage comme les points d'identification dans le premier état, et la détermination des points d'identification qui ne satisfont pas l'exigence de balayage comme les points d'identification dans le deuxième état comprend :
déterminer si les différents points d'identification correspondent à des points d'identification dans une base de données ;
déterminer les points d'identification qui sont appariés avec succès comme les points d'identification dans le premier état ; et
déterminer les points d'identification qui ne sont pas appariés avec succès comme les états d'identification dans le deuxième état.

8. Procédé selon l'une quelconque des revendications 3 à 7, dans lequel la détermination si les différents points d'identification satisfont l'exigence de balayage, la détermination des points d'identification qui satisfont l'exigence de balayage comme les points d'identification dans le premier état, et la détermination des points d'identification qui ne satisfont pas l'exigence de balayage comme les points d'identification dans le deuxième état comprend :
déterminer si les différents points d'identification sont situés dans une boîte de balayage ;
déterminer les points d'identification qui sont situés dans la boîte de balayage comme les points d'identification dans le premier état ; et
déterminer les points d'identification qui sont situés en dehors de la boîte de balayage comme les états d'identification dans le deuxième état.

9. Procédé selon l'une quelconque des revendications 3 à 8, dans lequel la détermination si les différents points d'identification satisfont l'exigence de balayage, la détermination des points d'identification qui satisfont l'exigence de balayage comme les points d'identification dans le premier état, et la détermination des points d'identification qui ne satisfont pas l'exigence de balayage comme les points d'identification dans le deuxième état comprend :
effectuer des opérations d'enregistrement entre les différents points d'identification et un modèle polyvalent correspondant aux différents points d'identification à des intervalles de temps prédéfinis ou après un nombre de balayage prédéterminé ;
déterminer si les différents points d'identification dans des modèles polyvalents enregistrés correspondent à des points d'identification dans la base de données ;
déterminer les points d'identification appariés comme les points d'identification dans le premier état, et déterminer les points d'identification non appariés comme les points d'identification dans le deuxième état.

10. Procédé selon l'une quelconque des revendications précédentes, dans lequel la détermination des informations d'état des différents points d'identification comprend :
déterminer si les nombres de fois de balayage des différents points d'identification satisfont un seuil de nombre de fois de balayage, et déterminer si les points d'identification, dont les nombres de fois de balayage atteignent le seuil de nombre de fois de balayage, correspondent à des points d'identification dans une base de données, et déterminer si les différents points d'identification sont situés dans une boîte de balayage ; et
déterminer les points d'identification qui sont situés dans la boîte de balayage et qui sont appariés avec succès, dont les nombres de fois de balayage atteignent le seuil de nombre de fois de balayage, comme les points d'identification dans un premier état ;
déterminer les points d'identification qui sont situés en dehors de la boîte de balayage et qui sont appariés avec succès, dont les nombres de fois de balayage atteignent le seuil de nombre de fois de balayage, comme les points d'identification dans un deuxième état ; et
déterminer les points d'identification qui ne sont pas appariés avec succès, dont les nombres de fois de balayage atteignent le seuil de nombre de fois de balayage, ou les points d'identification, dont les nombres de fois de balayage ne satisfont pas le seuil de nombre de fois de balayage, comme les points d'identification dans un troisième état.

11. Procédé selon la revendication 10, dans lequel l'affichage des différents points d'identification en fonction des informations d'état des différents points d'identification comprend :
afficher les points d'identification dans le premier état dans un premier mode d'affichage, afficher les points d'identification dans le deuxième état dans un deuxième mode d'affichage, et afficher les points d'identification dans un troisième état dans un troisième mode d'affichage, le premier mode d'affichage, le deuxième mode d'affichage et le troisième mode d'affichage étant différents les uns des autres ; et lorsque les différents points d'identification sont affichés, les modèles de tige de balayage correspondant aux différents points d'identification ne sont pas affichés.

12. Procédé selon l'une quelconque des revendications 3 à 11, dans lequel le procédé comprend en outre :
déterminer si les différents points d'identification correspondent à des points d'identification dans une base de données ;
calculer un pourcentage de points d'identification appariés avec succès par rapport à un nombre total de points d'identification dans la même zone balayée stockée dans la base de données ;
dans un cas où le pourcentage est inférieur à un seuil de pourcentage prédéterminé, ajouter un indicateur de guidage pour la même zone balayée ; dans un cas où l'indicateur de guidage indique que la zone balayée nécessite des balayages supplémentaires, afficher la même zone de balayage dans un quatrième état.

13. Procédé selon l'une quelconque des revendications précédentes, dans lequel le procédé comprend en outre :
faire correspondre les différents points d'identification avec des points d'identification des tiges de balayage dans une base de données standard ; et
afficher les points d'identification d'une tige de balayage appariée avec succès dans la base de données standard.

14. Procédé selon l'une quelconque des revendications précédentes, dans lequel le procédé comprend :
aligner les différents points d'identification avec un modèle polyvalent dans une base de données polyvalente pour obtenir un modèle cible ; et afficher le modèle cible, le modèle polyvalent comprenant des modèles tridimensionnels d'autres tiges de balayage.

15. Appareil d'affichage de données de balayage, appliqué à une scène de balayage avec une tige de balayage, ****caractérisé en ce qu'****il comprend :
un composant d'identification, configuré pour : dans la scène de balayage, identifier des points d'identification de la tige de balayage et déterminer des informations d'état des différents points d'identification ; et un premier composant d'affichage, configuré pour afficher les différents points d'identification en fonction des informations d'état des différents points d'identification.
